Europäisches Patentamt

European Patent Office

Office européen des brevets

(11) Numéro de publication: **0 307 626 B1**

(12) **FASCICULE DE BREVET EUROPEEN**

(45) Date de publication de fascicule du brevet: **06.05.92** (51) Int. Cl.5: **A23L 1/221**, A23L 3/34

(21) Numéro de dépôt: **88113185.8**

(22) Date de dépôt: **13.08.88**

(54) **Procédé d'obention d'un extrait antioxydant d'épices.**

(30) Priorité: **16.09.87 CH 3574/87**

(43) Date de publication de la demande:
**22.03.89 Bulletin 89/12**

(45) Mention de la délivrance du brevet:
**06.05.92 Bulletin 92/19**

(84) Etats contractants désignés:
**AT BE CH DE ES FR GB GR IT LI LU NL SE**

(56) Documents cités:
EP-A- 0 038 959      FR-A- 2 192 852
FR-A- 2 249 155      GB-A- 2 184 341
US-A- 3 950 226      US-A- 4 450 097

(73) Titulaire: **SOCIETE DES PRODUITS NESTLE S.A.**
**Case postale 353**
**CH-1800 Vevey(CH)**

(72) Inventeur: **Aeschbach, Robert**
**Bld. de Charmontey 4**
**CH-1800 Vevey(CH)**
Inventeur: **Philippossian, Georges**
**18 Av. de Valmont**
**CH-1010 Lausanne(CH)**

Rank Xerox (UK) Business Services
(3.08/2.18/2.0)

**Description**

L'invention concerne un procédé d'obtention d'un extrait antioxydant d'épices pratiquement sans odeur et sans goût, ainsi que l'utilisation dudit extraits.

Il est connu que certaines épices, telles que romarin et sauge ont une teneur élevée en antioxydants. On a déjà développé selon l'état de la technique des procédés permettant de débarasser lesdites épices de tous les composants ne participants pas à l'activité antioxydante. Le brevet EP 38 959 concerne un procédé de préparation de conservateurs ayant une action antioxydante et anti-bactérienne, dans lequel on soumet la matière de base, telle que romarin, à une extraction avec un mélange de solvant polaire et non polaire, par exemple de l'éthanol et du n-hexane, de manière à obtenir l'extrait d'épices prêt à l'emploi. L'inconvénient de ce procédé est qu'il ne permet pas d'obtenir un extrait d'épices totalement débarassé de son amertume et de toutes ses substances odorifiques. La même chose peut être dite pour le brevet GB 2184341 qui concerne un procédé d'extraction de romarin et sauge, dans lequel on extrait les matières antioxydantes aussi bien avec des solvants non polaires que polaires. Le brevet FR 2192852 concerne un procédé d'extraction d'arômes et parfums et non d'antioxydants, dans lequel on extrait la matière végétale avec un hydrocarbure fluoré non polaire et simultanément ou successivement avec un solvant polaire. Le brevet US 4450097 concerne un procédé d'obtention d'un antioxydant à partir de romarin, dans lequel on extrait ladite épice avec un solvant non polaire, on soumet l'extait à une distillation à la vapeur et on extrait le résidu ainsi obtenu avec une solution alcaline aqueuse ayant un pH d'au moins 10.5. L'inconvénient de ce procédé est qu'il ne permet pas d'extraire toute la matière antioxydante du romarin.

Le but du procédé selon la présente invention est de préparer un extrait d'épices sans odeur et sans goût de manière à pouvoir envisager une utilisation la plus large possible en mettant à profit le caractère de conservateur dudit extrait d'épices.

La présente invention concerne un procédé d'obtention d'un extrait antioxydant d'épices pratiquement sans odeur et sans goût, dans lequel on soumet ladite épice à au moins une extraction avec un solvant non polaire, on soumet l'épice ainsi traitée à au moins une extraction avec un solvant polaire et on concentre et sèche l'extrait avec le solvant polaire pour obtenir l'extrait d'épices.

Dans une forme de réalisation permettant d'augmenter le taux d'extraction de matières antioxydantes, on peut envisager de faire un traitement de l'extrait obtenu avec le solvant non polaire; dans ce cas, on mélange ledit extrait avec une solution aqueuse basique, on récupère la phase aqueuse qu'on acidifie et qu'on extrait au dichlorométhane et on mélange l'extrait obtenu avec la phase obtenue à partir de l'extraction avec le solvant polaire, on évapore et sèche le mélange de manière à obtenir un extrait d'épices à haute teneur en matière antioxydante. Le traitement de l'épice avec un solvant non polaire permet d'extraire pratiquement tous les constituants odorifiques de ladite épice. Le traitement subséquent avec une base de l'extrait non polaire permet de récupérer les composants antioxydants. La base utilisée est de préférence l'hydroxyde de sodium. L'extraction de l'épice avec un solvant polaire permet de faire passer dans la phase polaire tout le reste des composants responsables du caractère antioxydant.

Dans cette forme du procédé selon la présente invention, on obtient un extrait quasi dépourvu d'odeur et de couleur qui contient la totalité des constituants antioxydants disponibles dans l'épice et ceci par le fait que l'on reconstitue un produit antioxydant à partir de deux extraits qui ont eux-même des propriétés antioxydantes.

Par solvant non polaire, on entend les hydrocarbures saturés en $C_5$-$C_8$, ramifiés ou non, cyclisés ou non, y compris leurs mélanges, les hydrocarbures aromatiques volatils et les solvants chlorés, et par solant polaire les alcools en $C_1$-$C_4$, les cétones volatiles, l'acétone, la méthyl-éthyl cétone et les solvants du type éthers et esters. On utilise de préférence comme solvant non polaire l'hexane et comme solvant polaire l'éthanol.

Par épice soumise au traitement selon l'invention, on entend aussi bien l'épice entière que des résidus d'épice, c'est-à-dire des épices ayant subi une distillation à la vapeur d'eau.

Selon le produit de départ et le type d'utilisation finale on effectue entre une et sept extractions à l'hexane, de même que entre une et sept extractions à l'éthanol. Ces extractions se font normalement entre la température ambiante et le point d'ébullition du solvant, de préférence à une température comprise entre 20 et 30°C avec un rapport épice/hexane et épice/éthanol pour chaque extraction compris entre 1:1 et 1:10 ( poids/volume). Les extractions aussi bien à l'hexane qu'à l'éthanol se font en une durée comprise entre 30 minutes et 3 heures.

De manière à ne pas dégrader les substances antioxydantes, on effectue ces extractions en présence d'un gaz inerte, par exemple l'azote.

2

Le procédé selon l'invention permet d'obtenir un extrait d'épices avec une forte teneur en matière antioxydante. Cet extrait est cependant vert. Il est parfois intéressant, selon le type d'utilisation, de préparer un extrait d'épices incolore. Dans ce cas, après la dernière extraction à l'éthanol, on effectue une décoloration sur du charbon actif. Cette décoloration est faite avec une teneur en charbon actif comprise entre 10 et 20% en poids par rapport au produit final. On constate que le pouvoir antioxydant est moins marqué après la décoloration que sur un extrait d'épices non décoloré. Finalement, il reste à concentrer et à sécher la phase éthanolique : on effectue ce séchage de préférence sous vide pendant une dizaine d'heures à une température inférieure à 60°C de manière à ne pas détériorer les antioxydants présents. Selon une variante, on ajoute de l'eau à la solution éthanolique concentrée et on effectue un séchage par atomisation, de manière à obtenir une poudre fine de l'extrait d'épice. Les épices traitées selon l'invention sont le romarin et la sauge. Comme la poudre que l'on obtient selon le procédé de l'invention n'a pratiquement pas d'odeur et de goût, on peut en envisager tout type d'utilisation aussi bien dans le domaine alimentaire, que cosmétique et pharmaceutique. On l'incorpore à raison de 0,01 à 0,5% en poids par rapport au poids du produit à stabiliser.

Le procédé selon l'invention est mis en oeuvre soit en continu, par charges, à contre-courant ou en percolation.

La suite de la description est faite en référence aux exemples.

## Exemple 1 : Procédé par charges

500 g de résidu de romarin (feuilles de romarin ayant subi une distillation à la vapeur d'eau) sont extraites à température ambiante pendant 2,5 h avec 2,5 l d'hexane dans une cuve avec brasseur. On filtre et on répète l'extraction une deuxième fois dans les mêmes conditions. Les deux filtrats sont réunis et concentrés à un volume de 400 ml. Cet extrait à l'hexane est prudemment remué sous un léger courant d'azote avec 400 ml d'une solution aqueuse d'hydroxyde de sodium (2-normale). Après 10 minutes, la phase aqueuse est séparée de la phase organique et aussitôt acidifiée avec de l'acide sulfurique concentrée et ensuite extraite au dichlorométhane pour récupérer la matière antioxydante. On répète ce traitement de l'extrait à l'hexane une deuxième fois dans les mêmes conditions et on réunit les deux extraits au dichlorométhane qui en résultent.

Le reste de résidu de romarin, traité à l'hexane comme décrit ci-dessus est extrait sous brassage et sous azote à température ambiante pendant 2,5 h avec 2,5 l d'éthanol. On filtre et on décolore l'extrait alcoolique avec 5 g de charbon actif sous brassage et sous azote pendant 2,5 h à température ambiante. Après filtration sur célite, cet extrait est réuni avec l'extrait au dichlorométhane de la fraction non polaire et ce mélange est concentré à l'évaporateur rotatif et finalement évaporé à sec dans une étuve à vide. On obtient 57 g de poudre d'extrait de romarin (rendement : 11,4 %).

## Exemple 2

On opère comme dans l'exemple 1, mais sans décoloration de l'extrait éthanolique au charbon actif. L'extrait de romarin qui en résulte correspond à un rendement de 12,4%.

## Exemple 3 : Procédé par charges

On mélange dans une cuve avec brasseur, 1 kg de résidu de romarin moulu avec 5 l d'hexane. On extrait à température ambiante pendant 2,5 h sous azote et on filtre. Onmet au rebut la phase hexane et on répète l'opération dans les mêmes conditions que précédemment. Le résidu ainsi récupéré est mélangé à 5 l d'éthanol mélangé à 5% de méthanol. On effectue une extraction sous brassage pendant 2,5 h à température ambiante sous azote et on filtre de manière à récupérer 4,7 l d'alcool contenant les matières antioxydantes. On décolore ensuite sur 20 g de charbon actif pendant 2,5 h à température ambiante sous azote. Après filtration, il reste 4,6 litres d'éthanol. On effectue une concentration à l'évaporateur rotatif jusque 0,4 l et finalement une évaporation à sec sous vide (16.102 Pa ou 12 mm Hg) pendant 15 h à 50°C. On obtient 96,5 g de poudre d'extrait de romarin (rendement 9,65%).

## Exemple 4 : Procédé à contre-courant

On mélange 1 l d'hexane avec 1 kg de résidu de romarin moulu. On fait passer sur ce mélange 4 l d'hexane ayant déjà extrait deux autres charges de romarin et contenant 21 g de cires. Ces 4 l d'hexane contiennent après cette extraction 47 g de cires. On fait une seconde extraction avec 4 l d'hexane ayantdéjà extrait une autre charge de romarin et contenant 8 g de cires. A la sortie, ce volume d'hexane contient 21 g de cires. On fait une troisième extraction avec 4 l d'hexane frais contenant à la sortie 8 g de cires. On laisse sécher le résidu de romarin et on le mélange avec 1 l d'éthanol. On fait passer sur ce mélange 3 l d'éthanol ayant déjà extrait une autre charge de romarin et contenant avant passage 45 g et après 105 g d'extrait de romarin. On fait une deuxième extraction avec 3 l d'éthanol frais contenant à la sortie 45 g d'extrait brut. Le romarin ainsi extrait est mis au rebut. Avec les 3 l d'éthanol contenant 105 g d'extrait, on fait une décoloration sur charbon actif, on filtre, on concentre et on évapore de manière à obtenir 95 g de poudre d'extrait de romarin prêt à l'emploi (rendement 9,5 %).

Exemple 5 : Procédé par percolation

500 g de résidu de romarin sont disposés dans une colonne de chromatographie. On ajoute 1250 ml d'hexane et on extrait par percolation en faisant recirculer l'hexane dans la colonne pendant une demi-heure (débit: 750 ml/h.). L'hexane contenant les cires est ensuite drainé durant 15 minutes et les 750 ml d'extrait qui en résultent sont remplacés par de l'hexane frais. On répète l'opération sur la même colonne 6 fois. On laisse sécher le résidu sous un léger courant d'azote et on fait sur la même colonne une extraction par percolation en circuit avec 1250 ml d'éthanol pendant 30 minutes à température ambiante. L'éthanol est ensuite drainé durant 15 minutes et les 750 ml d'extrait ainsi obtenus sont remplacés par de l'éthanol frais. On répète six fois cette extraction à l'éthanol. On rassemble les 7 extraits d'éthanol, on concentre à volume moitié, on décolore pendant 2,5 h sur du charbon actif et après filtration on sèche sous vide. L'extrait de romarin obtenu (52,5 g de poudre sèche) correspond à un rendement de 10,5 %.

Exemple 6

On opère comme dans l'exemple 3, sans décoloration de l'extrait éthanolique. Rendement: 10,5 %.

Exemple 7

On opère comme dans l'exemple 3, mais avec de la sauge (distillée à la vapeur d'eau) comme matière première. Rendement: 12,4 %.

Le tableau ci-dessous montre les rendements, efficacités antioxydantes et propriétés organoleptiques des extraits obtenus selon les exemples 1 à 7. Ces données sont comparées avec celles de trois extraits de résidu de romarin ou de sauge, qui ont été obtenus par simple extraction avec l'éthanol (rapport épice/éthanol de 1:5 (poids/volume)) à température ambiante pendant 2,5 h, avec ou sans décoloration.

## TABLEAU

| Extrait | Taux d'extrac. % poids | Index anti-oxydant (a) | Valeur anti-oxydante (b) | Odeur (c) | Amer-tume (c) | Couleur |
|---|---|---|---|---|---|---|
| Ex. 1 | 11.4 | 4,3 | 49,2 | – | – | beige |
| Ex. 2 | 12,7 | 4,9 | 62,2 | – | – | verdâtre |
| Ex. 3 | 9,65 | 3,7 | 35,7 | (+) | – | jaunâtre |
| Ex. 4 | 9,5 | 3,6 | 34,2 | – | – | beige |
| Ex. 5 | 10,5 | 3,5 | 36,75 | – | – | beige |
| Ex. 6 | 10,5 | 4,2 | 44,1 | – | – | verdâtre |
| Ex. 7 | 12,4 | 4,8 | 59,5 | + | + | vert |
| Extrait éthano-lique de résidu de romarin | 11,9 | 3,9 | 46,4 | +++ | ++ | vert foncé |
| Extrait éthano-lique de résidu de romarin décoloré | 10,1 | 3,6 | 36,35 | ++ | ++ | vert |
| Extrait éthano-lique de résidu de sauge | 14,2 | 4,4 | 62,5 | +++ | ++ | vert foncé |

(a)  Index antioxydant: Test Rancimat (500 ppm

d'extrait, graisse de

poule, 110°C, air: 201/h)

(b)  Valeur antioxydante:          Produit de TE x IA

TE = Taux d'extraction

IA = Index antioxydant

(c) Critères          -     pratiquement sans

d'avaluation              odeur/sans amertume

+     peu d'odeur/peu

d'amertume

++    odorifique/amer

+++   fortement odorifique/

très amer

**Revendications**

**1.**  Procédé d'obtention d'un extrait antioxydant d'épices pratiquement sans odeur et sans goût, caractérisé en ce qu'on soumet ladite épice à au moins une extraction avec un solvant non polaire, on soumet l'épice ainsi traitée à au moins une extraction avec un solvant polaire et on concentre et sèche l'extrait avec le solvant polaire pour obtenir l'extrait d'épices.

**2.**  Procédé selon la revendication 1, caractérisé en ce qu'on traite l'extrait obtenu avec le solvant non polaire avec une solution aqueuse basique, on récupère la phase aqueuse qu'on acidifie et qu'on extrait au dichlorométhane et on mélange l'extrait obtenu avec la phase obtenue à partir de l'extraction avec le solvant polaire.

**3.**  Procédé selon l'une des revendications 1 ou 2, caractérisé en ce que le solvant non polaire est l'hexane et le solvant polaire est l'éthanol.

**4.**  Procédé selon la revendication 3, caractérisé en ce qu'on effectue entre une et sept extractions à l'hexane.

**5.**  Procédé selon la revendication 3, caractérisé en ce qu'on effectue entre une et sept extractions à l'éthanol.

**6.**  Procédé selon l'une des revendications 4 ou 5, caractérisé en ce qu'on effectue les extractions à l'hexane et à l'éthanol à une température comprise entre 20 et 30°C.

**7.** Procédé selon l'un des revendications 4 à 6, caractérisé en ce qu'on effectue chaque extraction à l'hexane et à l'éthanol en une durée comprise entre 30 minutes et 3 heures.

**8.** Procédé selon l'une des revendications 4 à 7, caractérisé en ce qu'on effectue les extractions à l'hexane et à l'éthanol en présence d'un gaz inerte.

**9.** Procédé selon l'une des revendications 4 à 8, caractérisé en ce qu'on effectue chaque extraction avec un rapport en poids/volume épice/hexane et épice/éthanol compris entre 1:1 et 1:10.

**10.** Procédé selon l'une des revendications 1 à 9, caractérisé en ce que après la dernière extraction à l'éthanol on effectue une décoloration sur du charbon actif.

**11.** Procédé selon l'une des revendications 1 à 10, caractérisé en ce que les épices traitées sont choisies dans le groupe formé par la sauge et le romarin.

**12.** Utilisation de l'extrait antioxydant d'épices susceptible d'être obtenu par le procédé selon l'une des revendications 1 à 11, caractérisé en ce qu'on l'incorpore dans des produits alimentaires, cosmétiques ou pharmaceutiques à une teneur comprise entre 0,01 et 0,5% en poids.

**Claims**

**1.** A process for the production of a substantially odourless and neutral-tasting antioxidant spice extract, characterized in that the spice is subjected to at least one extraction with a non-polar solvent, the spice thus treated is subjected to at least one extraction with a polar solvent and the extract containing the polar solvent is concentrated and dried to obtain the spice extract.

**2.** A process as claimed in claim 1, characterized in that the extract obtained with the non-polar solvent is treated with a basic agueous solution, the agueous phase is recovered, acidified and extracted with dichloromethane and the extract obtained is mixed with the phase obtained from the extraction with the polar solvent.

**3.** A process as claimed in claim 1 or 2, characterized in that the non-polar solvent is hexane while the polar solvent is ethanol.

**4.** A process as claimed in claim 3, characterized in that between 1 and 7 extractions are carried out with hexane.

**5.** A process as claimed in claim 3, characterized in that between 1 and 7 extractions are carried out with ethanol.

**6.** A process as claimed in claim 4 or 5, characterized in that the extractions with hexane and ethanol are carried out at a temperature in the range from 20 to 30°C.

**7.** A process as claimed in any of claims 4 to 6, characterized in that each extraction with hexane and with ethanol is carried out over a period of from 30 minutes to 3 hours.

**8.** A process as claimed in any of claims 4 to 7, characterized in that the extractions with hexane and with ethanol are carried out in the presence of an inert gas.

**9.** A process as claimed in any of claims 4 to 8, characterized in that each extraction is carried out with a ratio by weight/volume of spice to hexane and spice to ethanol of from 1:1 to 1:10.

**10.** A process as claimed in any of claims 1 to 9, characterized in that the last extraction with ethanol is followed by decoloration with active carbon.

**11.** A process as claimed in any of claims 1 to 10, characterized in that the spices treated are selected from the group consisting of sage and rosemary.

7

**12.** The use of the antioxidant spice extract capable of being obtained by the process claimed in any of claims 1 to 11, characterized in that it is incorporated in food, cosmetic or pharmaceutical products in a content of from 0.01 to 0.5% by weight.

**Patentansprüche**

**1.** Verfahren zur Gewinnung eines als Oxidationsinhibitor wirksamen, praktisch geruch- und geschmacklosen Gewürzextraktes, dadurch gekeennzeichnet daß man das genannte Gewürz wenigstens einer Extraktion mit einem nicht-polaren Lösungsmittel unterwirft, daß man das so behandelte Gewürz wenigstens einer Extraktion mit einem polaren Lösungsmittel unterwirft, und daß man den Extrakt mit dem polaren Lösungsmittel konzentriert und trocknet, um den Gewürzextrakt zu gewinnen.

**2.** Verfahren nach Anspruch 1, dadurch gekennzeichnet, daß man den mit dem nicht-polaren Lösungsmittel erhaltenen Extrakt mit einer wässerigen, basischen Lösung behandelt, daß man die wässerige Phase gewinnt und ansäuert, daß man dieselbe mit Dichlormethan extrahiert, und daß man den erhaltenen Extrakt mit jener Phase vermischt, die aus der Extraktion mit dem polaren Lösungsmittel erhalten worden ist.

**3.** Verfahren nach Anspruch 1 oder 2, dadurch gekennzeichnet, daß das nicht-polare Lösungsmittel Hexan ist und daß das polare Lösungsmittel Ethanol ist.

**4.** Verfahren nach Anspruch 3, dadurch gekennzeichnet, daß man zwischen einer und sieben Extraktionen mit Hexan durchführt.

**5.** Verfahren nach Anspruch 3, dadurch gekennzeichnet, daß man zwischen einer und sieben Extraktionen mit Ethanol durchführt.

**6.** Verfahren nach Anspruch 4 oder 5, dadurch gekennzeichnet, daß man die Extraktionen mit Hexan bzw. mit Ethanol bei einer zwischen 20 und 30°C liegenden Temperatur durchführt.

**7.** Verfahren nach einem der Ansprüche 4 bis 6, dadurch gekennzeichnet, daß man jede Extraktion mit Hexan bzw. mit Ethanol während einer Zeitdauer durchführt, welche zwischen 30 min und 3 h liegt.

**8.** Verfahren nach einem der Ansprüche 4 bis 7, dadurch gekennzeichnet, daß man die Extraktionen mit Hexan bzw. mit Ethanol in Gegenwart eines inerten Gases durchführt.

**9.** Verfahren nach einer der Ansprüche 4 bis 8, dadurch gekennzeichnet, daß man jede Extraktion bei einem in Gew./Volumen ausgedrückten Verhältnis von Gewürz/Hexan bzw. Gewürz/Ethanol zwischen 1:1 und 1:10 durchführt.

**10.** Verfahren nach einem der Ansprüche 1 bis 9, dadurch gekennzeichnet, daß man nach der letzten Extraktion mit Ethanol ein Entfärben über Aktivkohle durchführt.

**11.** Verfahren nach einem der Ansprüche 1 bis 10, dadurch gekennzeichnet, daß die behandelten Gewürze aus der durch Salbei und Rosmarin gebildeten Gruppe ausgewählt sind.

**12.** Verwendung des als Oxidationsinhibitors wirksamen Gewürzextraktes, welcher nach dem Verfahren nach einem der Ansprüche 1 bis 11 erhältlich ist, dadurch gekennzeichnet, daß man diesen Gewürzextrakt in Nahrungsmittel, kosmetische oder pharmazeutische Produkte in einer Menge einverleibt, welche zwischen 0,01 und 0,5 Gew.-% liegt.